# EUROPEAN PATENT APPLICATION

(11) **EP 4 342 874 A1**
(43) Date of publication of application: **27.03.2024**
(21) Application number: 22196574.2
(22) Date of filing: 20.09.2022
(51) Int. Cl.: C07C 41/42, C07C 43/23, C07C 41/40

(54) **BIOBASED SURFACTANT AND ANTIOXIDANT**

(71) Applicant: Bloom Biorenewables SA, 1723 Marly (CH)
(72) Inventor: QUESTELL-SANTIAGO, Ydna, 1020 Renens (CH); BERNARDES FIGUEIRÊDO, Monique, 1008 Prilly (CH); CHARMILLOT, Justine, 1006 Lausanne (CH); WEGMANN, Chloé, 1003 Lausanne (CH)
(74) Representative: Schaad, Balass, Menzl & Partner AG

(57) **Abstract**

The invention relates to a method for isolating of a substituted guaiacol and/or a substituted syringol of formula (I) 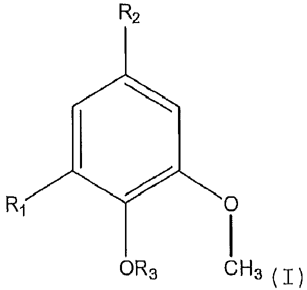 from a mixture containing lignin fragments, wherein
R₁ is hydrogen or -OCH₃;
R₂ is a linear or branched C₁ - C₄ alkyl or a linear or branched C₁ - C₄ hydroxyalkyl;
R₃ is hydrogen or Na;
R₄ is a C₁ - C₄ alkyl, a C₆ - C₁₂ aryl or a C₅ - C₁₂ cycloalkyl; comprising at least the steps of:
a) Providing a mixture containing lignin fragments;
b) Separation of the mixture of step a) into a lignin oligomer enriched fraction and a lignin monomer enriched fraction;
c) Separating a solution comprising the compound according to formula (I) by fraction distillation, wherein R₃ is hydrogen, from the lignin monomer enriched fraction of step b) ;
d) Adding an aqueous NaOH solution having a pH of 11 or more to the solution of step c) comprising compound according to formula (I), wherein R3 is a cation;
e) Isolating of the precipitated compound of step d); optionally followed by;
f) Dissolve the isolated compound of step e) in water and lowering the pH of the aqueous solution to a value below pH 10 using an acid;
g) Isolation of a compound according to formula (I), wherein R₃ is hydrogen, from the solution of step f) .

## Description

The present invention relates to a method for the isolation of compounds from lignin composition and their use as bio-based surfactant, antioxidants, UV-light absorber and stabilizing agent.

Surfactants, antioxidants and stabilizing agents are a class of chemicals used in a broad range of applications in the home care, personal care and agrochemical industries, in the food, medical and health sector, and in material formulations such as polymeric materials, fuels, engine oils, paint, ink, adhesives and waxes. Given their diverse market and large demand, it is of great interest that their production does not only rely on fossil-based sources, but sourced from renewable feedstocks with short lifetime (specially for surfactants) to minimize global environmental concerns. Natural antioxidants and bio-based surfactants are commercially available. Although, their performance is comparable or overpass fossil-based benchmarks, the production of these chemicals still pose an environmental pressure, due to inefficient extraction procedures or deforestation issues as they often require the use of food or virgin feedstocks (e.g. fruits or tropical plant fatty acids).

WO2018/134427A1. discloses the synthesis of alternative bisphenols using 4-alkylphenol derivatives (e.g. 2-methoxy-4-n-propylphenol and 2,6-dimethoxy-4-n-propylphenol). Obtained from the depolymerization of lignin enclosed in lignocellulosic biomass.

US2888503A discloses the usage of 2,6-dimethoxy-4-propylphenol as antiozidant in light-colored rubber for stabilization against cracking due to ozone.

Ren et al. (Green Chemistry 2022) disclose a method for the isolation of 4-propylsyringol and 4-propylguaiacol by extracting a monomer enriched oil from lignin oil with a 0.3 - 0,1 mol/L KOH solution. In a second step the extracting solution was rotary-evaporated to partially remove water to increase the KOH concentration to above 0.5ml/L. The KOH solution was cooled down to precipitate crystals. Further Ren et al. discloses an *in silico* experiment the distillation of a stream of a 4-propylsyringol-enriched solution and a 4-propylguaiacol-enriched solution using two distillation units affords 4-propylsyringol with 98.4% purity and 94.5% recovery, while all 4-propylguaiacol is recovered with 67.3% purity. A real distillation experiment is not disclosed due to the difficulty to prepare enough feedstock for distillation. Ren et al. also disclose that a NaOH solution is not suitable for purifying of 4-propylsyringol by isolating 4-propylsyringol sodium salt.

The problem of the present invention is therefore to provide a method for isolating a bio-based compound with surfactant, antioxidative, UV-light absorbing and stabilizing properties in a few steps directly from sustainable sources.

The problem is solved by the method according to claim 1. Further preferred embodiments are subject of the dependent claims.

The present invention relates to a method for isolating a substituted guaiacol and/or a substituted syringol of the general formula (I) 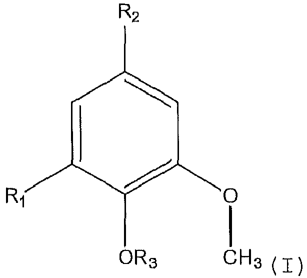 from a mixture containing lignin fragments,
wherein R₁ is hydrogen or -OCH₃ and
wherein R₂ is a linear or branched C₁ - C₄ alkyl or a linear or branched C₁ - C₄ hydroxyalkyl and
wherein R₃ is hydrogen or Na and
R₄ is a C₁ - C₄ alkyl, a C₆ - C₁₂ aryl or a C₅ - C₁₂ cycloalkyl.

The method comprises at least the steps of first providing a mixture containing lignin fragments. In the context of this invention the term "mixture" can be also understood as a solution or composition. Second, the mixture of the first step is separated into a lignin oligomer enriched fraction and a lignin monomer enriched fraction. Third, a solution comprising the compound according to formula (I), wherein R₃ is hydrogen, is separated from the lignin monomer enriched fraction of the second step. Fourth, an aqueous NaOH solution having a pH of 11 or more is added to the solution of the third step to initiate the precipitation of the compound according to formula (I). After the precipitation the solution comprises the compound according to formula (I), wherein R₃ is a cation. In a fifth step the precipitated compound of the fourth step is isolated.

The method according to the invention comprises the following optional steps six and seven. In a sixth step the isolated compound of the fifth step is dissolved in water and the pH of the aqueous solution is lowered to a value below pH 10 using an acid. In a seventh step a compound according to formula (I), wherein R₃ is hydrogen is isolated from the solution of step six.

Preferably the composition containing lignin fragments is obtained by reductive depolymerization of lignin.

In a preferred embodiment of the invention the lignin oligomer enriched fraction and the lignin monomer enriched fraction are separated in the second step either by distillation, at least one membrane or liquid-liquid extraction using a solvent of low polarity such as diethyl ether. In distillation, the light lignin fragments and other small fragments are evaporated from the solution and the remaining fraction is recovered as powder containing heavier lignin fragments (such as lignin dimers and oligomers). In membrane separation, the small lignin fragments and other small mixture components are ultra-filtered from the large lignin fragments by size exclusion, which are enriched in lignin oligomers. In liquid-liquid extraction, the light phase containing lignin monomers and other small fragments are solubilized and the remaining fraction is enriched in heavier fragments such as lignin oligomers.

The isolation of the compounds according to formula (I) in the third step is performed by fraction distillation. In the fraction distillation, the evaporation selectivity is driven by changes in pressure and/or temperature where the column consists of a stripping and/or rectifying section.

Preferably, the NaOH solution of the fourth step comprising the compound according to formula (I) has a pH of 12 to 13.

In the fifth step it is preferred that an emulsion is formed when the pH is lowered. Preferably the heavier phase of the emulsion comprises the lignin monomers, which can be separated by decantation or centrifugation.

It has surprisingly been found that the method for isolating of a substituted guaiacol and/or a substituted syringol of formula (I) resulted in the production of these compounds in a high yield despite using a fraction distillation for separating the compounds of formula (I) from the lignin monomer enriched fraction and using an NaOH solution for precipitation. The method according to the invention is easy to handle, easy to control and enables an industrial scale-up to produce inexpensive and high-quality compounds. Further the method according to the invention enables a continuous production of the compounds to be isolated. An additional benefit is that with the method of the invention the mixtures comprising the compounds to be isolated do not contain sugars, which highly facilitates the further processing and avoids the formation of humins and other degradation products.

In a preferred embodiment of the invention R₁ is hydrogen.

In another preferred embodiment of the invention R₁ is -OCH₃.

Preferably R₂ is selected from the group consisting of -(CH₂)₂-CH₃, -(CH₂)-CH₃, -CH₃ and -(CH₂)₃-OH.

In a preferred embodiment of the invention the compound of formula (I) is selected from the group consisting of

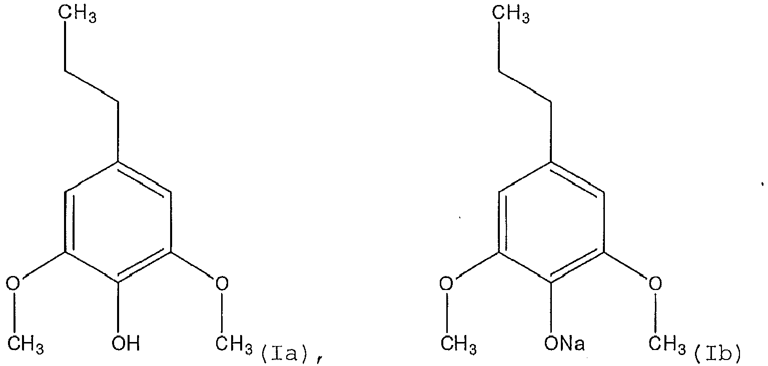

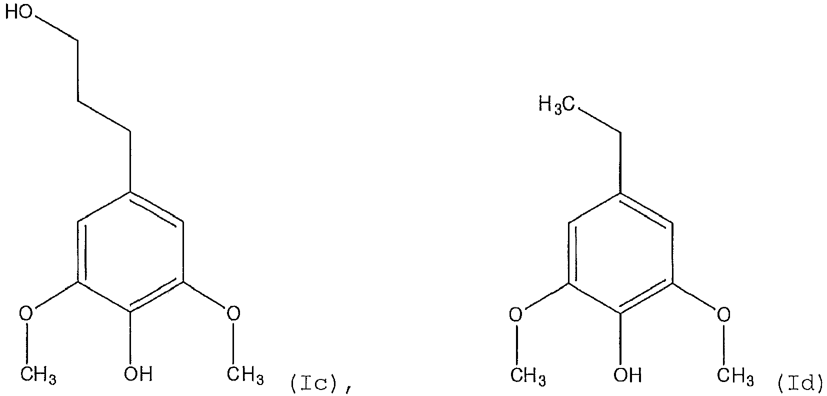

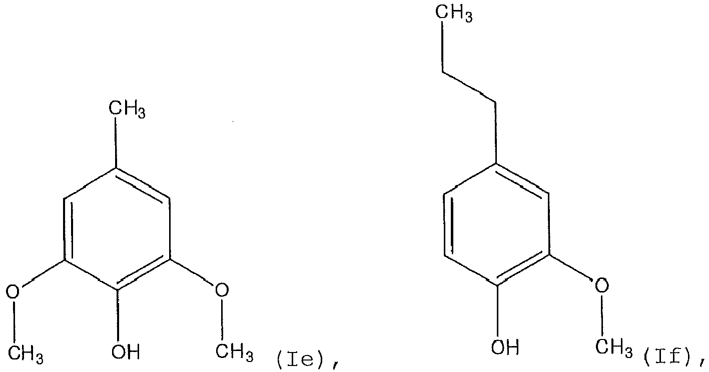

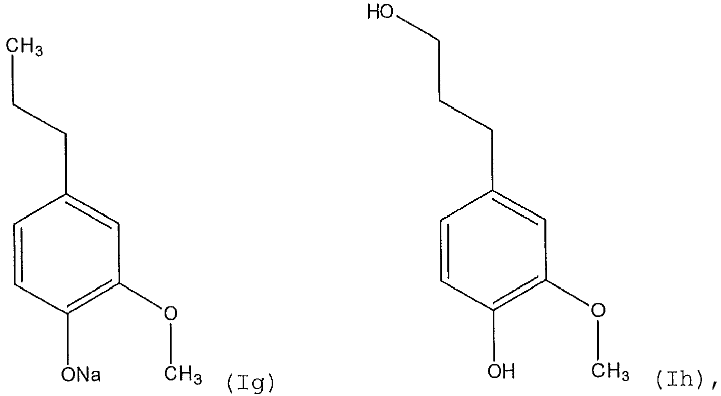

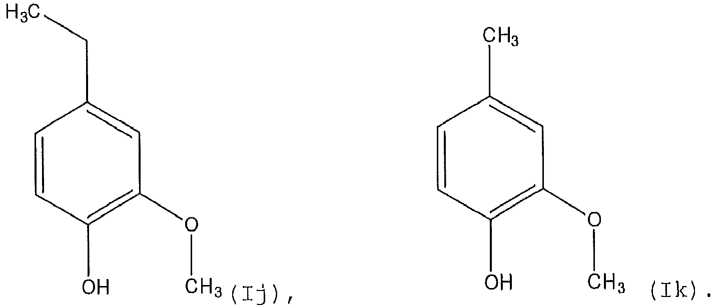

In a preferred embodiment of the invention the concentration of NaOH in the aqueous alkaline solution is 0.1 to 4 mol/L.

Preferably the precipitation is performed at room temperature.

In a preferred embodiment of the invention the method comprises the following subsequent steps six and seven. In a sixth step the isolated compound of the fifth step is dissolved in water and the pH of the aqueous solution is lowered to a value below pH 11 using an acid. In a seventh step a compound according to formula (I), wherein R₃ is hydrogen is isolated from the solution of step six.

Method according to Claim 9, wherein in step f) the pH is adjusted to 4 to 7.

Method according to Claims 9 or 10, wherein in step f) the acid is HCl, p-TSA, acetic caid, H₃PO₄ or H₂SO₄.

The present invention also encompasses a composition comprising a compound according to formular (I) as mentioned above.

The present invention also encompasses the use of the composition as mentioned above as a surfactant, antioxidant, UV-light absorber or stabilizing agent.

Preferably the composition comprises 4-propylsyringol as a surfactant, UV light absorber or stabilizing agent.

Preferably the composition comprises 4-propylsyringol salt as a surfactant, antioxidant, UV light absorber or stabilizing agent.

A preferred embodiment of the invention is the use of 4-propylsyringol or 4-propylsyringol salt as a UV-light absorber for coatings for example in sunglasses or as an ingredient in sun creams.

Another preferred embodiment of the invention is the use of 4-propylsyringol or 4-propylsyringol salt as a stabilizing agent for emulsion stability. These compounds have the same applications as surfactants to minimize coalescence of colliding droplets for examples as active pharma ingredients, food products such as ice cream or milk and in cosmetic products.

The present invention also encompasses the use of 4-propylsyringol as a non-ionic surfactant.

The present invention further encompasses the use of 4-propylsyringol salt as an ionic surfactant.

The present invention further encompasses the use of 4-propylsyringol and/or 4-propylsyringol salt as surfactant.

Preferably 4-propylsyringol and 4-propylsyringol salt are used as surfactants in the baking industry, pulp and paper processing, agrochemical formulations, firefighting applications, pipelines, personal care products, cleaning products, petrochemical, medical and pharmaceutical products such as toothpaste, detergents, cosmetics, paints, adhesives, inks, waxes, laxatives and food.

The present invention also encompasses the use of 4-propylguaiacols, 4-propanolsyringol, 4-propanolguaicaol and combinations thereof as antioxidant.

The present invention also encompasses the use of 4-propylguaiacol, 4-propylsyringol and combinations thereof as UV-light absorber.

The present invention also encompasses the use of 4-propylguaiacol, 4-propylsyringol and combinations thereof as stabilizing agent.

The present invention also encompasses a composition comprising at least the surface-active components 4-propylsyringol and a 4-propylsyringol salt.

The present invention also encompasses a composition comprising at least the surface-active components 4-propanolsyringol and 4-propanolguaiacol.

### Experimental data

### Separation of lignin monomers and lignin oligomers from lignin oil

The setup consists of 2 flasks (lignin mixture container and fraction collector) connected by a glass bridge. The collector is connected to vacuum line and cooled down using liquid nitrogen. After assembling the setup, the system is evacuated by vacuum and the lignin mixture flask is heated to the desired temperature accordingly to the fraction number to be collected and stirred using a stirring and heating plate. Each fraction is collected after the other by modifying the vacuum and the temperature (Fraction 0 at 50°C and 0.7 mbar; Fraction 1 at 210 °C using a ramp of 1 °C/min starting at 50 °C and 0.2 mbar). After all volatiles are out, the heavier lignin fragments (Fraction oligomers) are recovered after being cool down to room temperature.

**Table 1**

| Grams | Start. | Frac. 0 | Frac. 1 | Frac. oligo |
|---|---|---|---|---|
| 4-methylguaiacol | 0 | 0 | 1 | 0 |
| 4-ethylguaiacol | 1 | 0 | 1 | 0 |
| 4-propylguaiacol | 54 | 0 | 59 | 0 |
| 4-propanolguaiacol | 6 | 0 | 5 | 0 |
| 4-methylsyringol | 0 | 0 | 0 | 0 |
| 4-ethylsyringol | 1 | 0 | 2 | 0 |
| 4-propylsyringol | 102 | 0 | 111 | 0 |
| 4-propanolsyringol | 16 | 0 | 8 | 6 |
| Eugenol | 1 | 0 | 1 | 0 |
| Isoeugenol | 1 | 0 | 1 | 0 |
| Methoxyisoeugenol | 9 | 0 | 10 | 0 |
| Buthylated hydroxytoluene | 4 | 0 | 4 | 0 |
| Ethanol | 2 | 1 | 7 | 0 |
| Ethyl glycolate | 82 | 24 | 68 | 0 |
| Glycolic acid | 24 | 0 | 28 | 0 |
| Dimers + oligomers + unknown | 498 | 0 | 0 | 0 |
| Dimers + unknown | 0 | 5 | 32 | 0 |
| Dimers + oligomers | 0 | 0 | 0 | 423 |
| Total | 801 | 31 | 339 | 430 |

**Table 2**

| wt% | Start. | Frac, 0 | Frac, 1 | Frac. oligo |
|---|---|---|---|---|
| 4-methylguaiacol | 0.0 | 0.0 | 0.2 | 0 |
| 4-ethylguaiacol | 0.1 | 0.0 | 0.2 | 0 |
| 4-propylguaiacol | 6.7 | 1.5 | 17.3 | 0 |
| 4-propanolguaiacol | 0.8 | 0.0 | 1.5 | 0 |
| 4-methylsyringol | 0.0 | 0.0 | 0.0 | 0 |
| 4-ethylsyringol | 0.2 | 0.0 | 0.4 | 0 |
| 4-propylsyringol | 12.7 | 0.0 | 32.9 | 0 |
| 4-propanolsyringol | 2.0 | 0.0 | 2.4 | 1 |
| Eugenol | 0.1 | 0.0 | 0.3 | 0 |
| Isoeugenol | 0.1 | 0.0 | 0.4 | 0 |
| Methoxyisoeugenol | 1.2 | 0.0 | 3.0 | 0 |
| Buthylated hydroxytoluene | 0.5 | 0.3 | 1.2 | 0 |
| Ethanol | 0.3 | 4.8 | 2.2 | 0 |
| Ethyl glycolate | 10.2 | 75.9 | 20.1 | 0 |
| Glycolic acid | 2.9 | 0.0 | 8.3 | 0 |
| Dimers + oligomers + unknown | 62.0 | 0.0 | 0.0 | 0 |
| Dimers + unknown | nd | 0.0 | 9.6 | 0 |
| Dimers + oligomers | nd | 0.0 | 0.0 | 98.4 |
| Total | 99.8 | 82.5 | 100 | 99.4 |

### 5 Purification by fractional distillation

The setup for the fraction distillation consists of 2 flasks (lignin mixture container and fraction collector), a Vigreux column and a glass bridge. The mixture container is connected to the Vigreux column, and this to a glass bridge with the collector at the other end. The collector is connected to vacuum line and cooled down using liquid nitrogen. After assembling the setup, the system is evacuated by vacuum and the lignin mixture is heated and stirred using a stirring and heating plate. Each fraction is collected after the other by modifying the vacuum and the temperature (F0 at 135 °C and 1.0 mbar; F1 at 145 °C and 0.7 mbar; F2 at 150 °C and 0.6 mbar; F3 at 155 °C and 0.4 mbar; Fr are the residues of the distillation after the collection of F3.

**Table 3**

| wt% | F0 | F1 | F2 | F3 | Fr |
|---|---|---|---|---|---|
| 4-methylguaiacol | 1 | 0 | 0 | 0 | 0 |
| 4-ethylguaiacol | 0 | 0 | 0 | 0 | 0 |
| 4-propylguaiacol | 25 | 1 | 0 | 0 | 0 |
| 4-propanolguaiacol | 0 | 0 | 4 | 5 | 5 |
| 4-methylsyringol | 0 | 0 | 0 | 1 | 0 |
| 4-ethylsyringol | 1 | 4 | 1 | 0 | 0 |
| 4-propylsyringol | 6 | 73 | 89 | 67 | 7 |
| 4-propanolsyringol | 0 | 0 | 0 | 0 | 0 |
| Eugenol | 2 | 1 | 0 | 0 | 0 |
| Isoeugenol | 5 | 7 | 0 | 0 | 0 |
| Methoxyisoeugenol | 0 | 3 | 5 | 11 | 43 |
| Buthylated hydroxytoluene | 1 | 0 | 0 | 0 | 0 |
| Ethanol | 4 | 0 | 0 | 0 | 0 |
| Ethyl glycolate | 1 | 0 | 0 | 0 | 0 |
| Glycolic acid | 9 | 0 | 0 | 0 | 0 |
| Total | 55 | 89 | 99 | 84 | 56 |

### Purification by pH-driven precipitation method

The setup consists of a dropping funnel, one flask with a magnetic stir bar, a stirring plate, a Buchner filter, and an Erlenmeyer with vacuum connection. The base solution is added drop wise into the lignin monomer mixture while stirring. The precipitates are then filtered and dry at 50 °C and 100 mbar in a vacuum oven. Optionally, the remaining monomers in the aqueous liquid phase can be neutralized and extracted with ethyl acetate or hexanes to recover the other monomers.

**Table 4**

| wt% | Start. | Liquid organic phase | Precipitate salt |
|---|---|---|---|
| 4-methylguaiacol | 0.2 | 0.5 | 0.0 |
| 4-ethylguaiacol | 0.2 | 0.5 | 0.0 |
| 4-propylguaiacol | 17.3 | 53.1 | 5.2 |
| 4-propanolguaiacol | 1.5 | 0.2 | 0.0 |
| 4-methylsyringol | 0.0 | 0.0 | 0.0 |
| 4-ethylsyringol | 0.4 | 0.4 | 0.6 |
| 4-propylsyringol | 32.9 | 8.3 | 93.9 |
| 4-propanolsyringol | 2.4 | 2.1 | 1.1 |
| Eugenol | 0.3 | 0.4 | 0.0 |
| Isoeugenol | 0.4 | 1.1 | 0.0 |
| Methoxyisoeugenol | 3.0 | 5.6 | 2.9 |
| Buthylated hydroxytoluene | 1.2 | 0.4 | 0.2 |
| Ethanol | 2.2 | 0.2 | 0.0 |
| Ethyl glycolate | 20.1 | 0.0 | 0.0 |
| Glycolic acid | 8.3 | 0.0 | 0.0 |
| Total | 90.4 | 72.7 | 103.8 |

**NMR Analysis of solids obtained by pH-driven precipitation method using 1M NaOH and ddH₂O as solvent.**
4-propylsyringol sodium salt (Ib)
1H NMR (400 MHz, D2O) δ 6.48 (s, 1H), 3.68 (s, 3H), 2.39 (t, J = 7.6 Hz, 1H), 1.50 (h, J = 7.4 Hz, 1H), 0.82 (t, J = 7.4 Hz, 2H)

### Scale-up data for the pH driven precipitation of 4-propylsyringol salts

The setup consists of a dropping funnel, one flask with a magnetic stir bar, a stirring plate, a Buchner filter, and an Erlenmeyer with vacuum connection. The base solution is added drop wise into the lignin monomer mixture while stirring. The precipitates are then filtered and dry at 50°C and 100 mbar in a vacuum oven. Optionally, the remaining monomers in the aqueous liquid phase can be neutralized and extracted with ethyl acetate or hexanes to recover the other monomers.

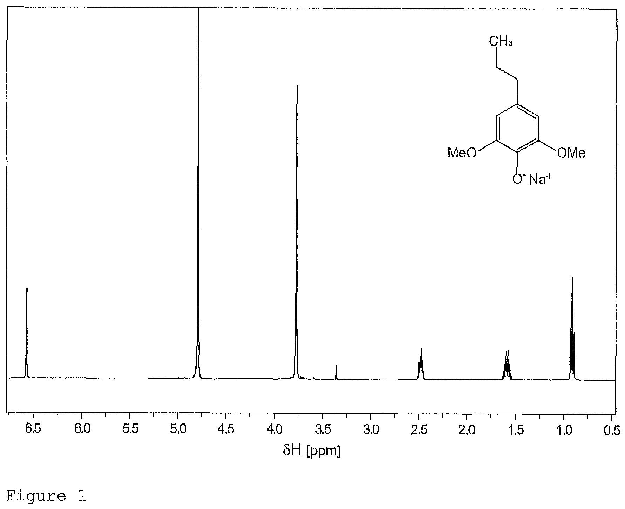

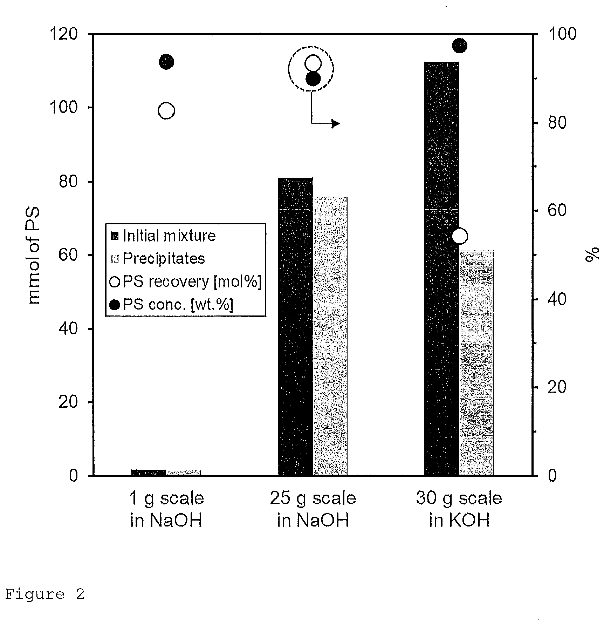

**Table 5: PS = 4-propylsyringol, data according to figure 2.**

| mmol of PS | 1g scale in NaOH | 25g scale in NaOH | 30g scale in KOH |
|---|---|---|---|
| Initial mixture | 1.7 | 81.1 | 112.7 |
| precipitates | 1.4 | 75.8 | 61.3 |
| PS recovery [mol%] | 82.9 | 93.5 | 54.4 |
| PS concentration [wt%] | 93.9 | 90.0 | 97.5 |

### Testing of 4-propylsyringol as antioxidant

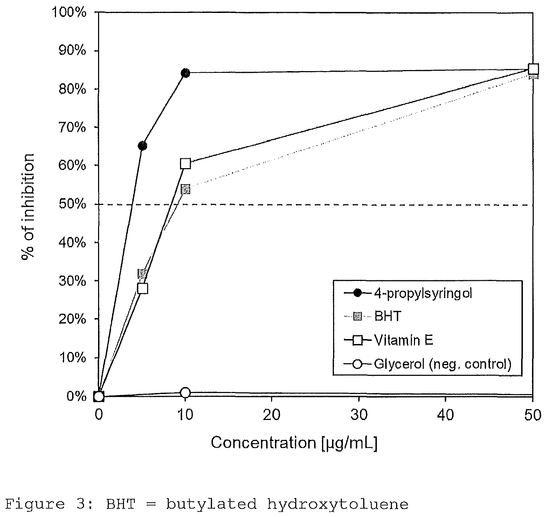

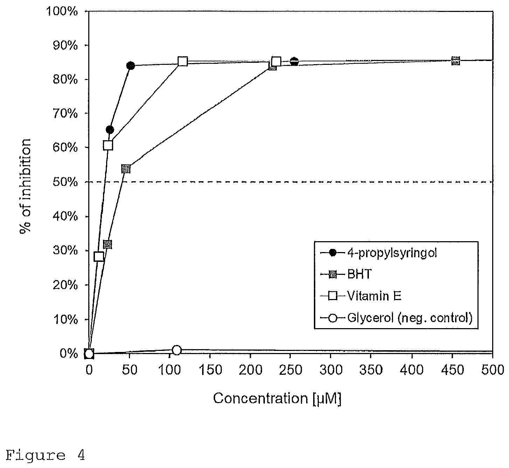

The antioxidant activity is assessed by measuring the capacity of the compound to react with a free radical (here 2,2-diphenyl-1-picrylhydrazyl also known as DPPH). The measurement is based on the color difference of the DPPH in solution upon the addition of an antioxidant at different compound composition and measured by UV/visible spectrophotometry within 400-700 nm range (517 nm is the preferred wavelength). Butylated hydroxytoluene (BHT) and vitamin E were used as refence substances (positive control) and glycerol as negative control (see figures 3 and 4). At a concentration of 5 pg/mL, 4-propylsyringol has an inhibition rate of 65%, while BHT and vitamin E have inhibition rates of 32% and 28%, respectively. At a concentration of 10 µg/mL, 4-propylsyringol has an inhibition rate of 84%, while BHT and vitamin E have inhibition rates of 54% and 61%, respectively. Only at a high concentration of 50 µg/mL, 4-propylsyringol BHT and vitamin E have similar inhibition rates of 85%, 85% and 84%, respectively (figure 3). This clearly demonstrates the superior activity of 4-propylsyringol as an antioxidant.

### Testing of 4-propylsyringol as surfactant

Surface tension measurements were performed using the pendant drop method by Krüss EasyDrop Standard drop shape analysis system. The measurements use the water/air interface to determine the surface tension of different compounds at application-relevant concentrations (0.1 to 1.0 wt%).

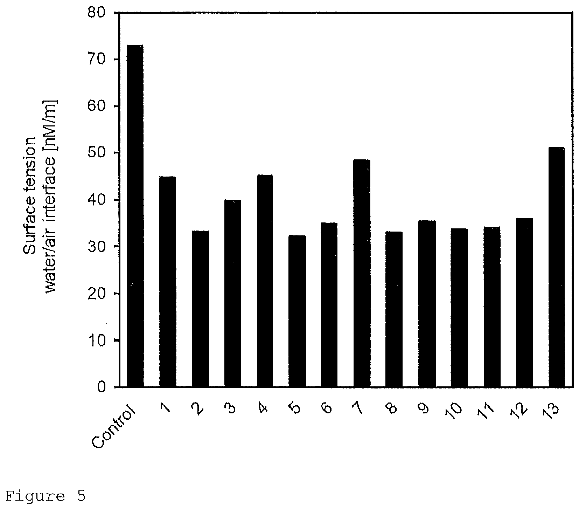

**Table 6: POHG = 4-propanolguaiacol, POHS = 4-propanolsyringol, PS = 4-propylsyringol, PSNa = 4-propylsyringol sodium salt, NaLAS = Sodium linear alkylbenzenesulfonate; SLS = sodium lauryl sulfate, SLES = sodium lauryl ether sulfate, SDBS = sodium dodecylbenzenesulfonate sodium, NPE = nonylphenol ethoxylate, DOSS = Dioctyl Sodium Sulfosuccinate; see figure 5. In sample 1 comprises 26wt% POHG, 61wt% POHS, 3wt% 4-proylsyringol, 0.6wt% 4-ethylsyringol and 0.3wt% methoxyisoeugenol.**

| Sample | Compound | Concentration [wt%] | Water/air interface [nM/m] |
|---|---|---|---|
| Control | None | 0.00 | 72.8 |
| 1 | POHG+POHS | 1.00 | 44.8 |
| 2 | PSNa | 1.00 | 33.8 |
| 3 | PSNa | 0.50 | 39.8 |
| 4 | PSNa | 0.10 | 45.1 |
| 5 | PS | 1.00 | 32.3 |
| 6 | PS | 0.50 | 34.9 |
| 7 | PS | 0.10 | 48.3 |
| 8 | NaLAS | 0.10 | 33.0 |
| 9 | SLS | 0.20 | 35.4 |
| 10 | SLES | 0.02 | 33.8 |
| 11 | SDBS | 0.01 | 34.1 |
| 12 | NPE | 0.02 | 36.0 |
| 13 | DOSS | 0.20 | 51.0 |

## Claims

1. Method for isolating of a substituted guaiacol and/or a substituted syringol of formula (I) 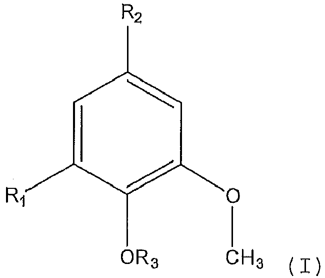 from a mixture containing lignin fragments,
wherein
R₁ is hydrogen or -OCH₃;
R₂ is a linear or branched C₁ - C₄ alkyl or a linear or branched C₁ - C₄ hydroxyalkyl;
R₃ is hydrogen or Na;
R4 is a C₁ - C₄ alkyl, a C₆ - C₁₂ aryl or a C₅ - C₁₂ cycloalkyl;
comprising at least the steps of:
a) Providing a mixture containing lignin fragments;
b) Separation of the mixture of step a) into a lignin oligomer enriched fraction and a lignin monomer enriched fraction;
c) Separating a solution comprising the compound according to formula (I) by fraction distillation, wherein R₃ is hydrogen, from the lignin monomer enriched fraction of step b);
d) Adding an aqueous NaOH solution to the solution of step c) comprising compound according to formula (I) to obtain a pH of at least 11, wherein R₃ is Na;
e) Isolating of the precipitated compound of step d); optionally followed by;
f) Dissolve the isolated compound of step e) in water and lowering the pH of the aqueous solution to a value below pH 11 using an acid;
g) Isolation of a compound according to formula (I), wherein R₃ is hydrogen, from the solution of step f) .

2. Method according to Claim 1, wherein R₁ is hydrogen.

3. Method according to Claim 1, wherein R₁ is -OCH₃.

4. Method according to Claim 2 or 3, wherein R₂ is selected from the group consisting of -(CH₂)₂-CH₃, -(CH₂)-CH₃, -CH₃ and -(CH₂)₃-OH.

5. Method according to one of the Claims 1 to 4, wherein the concentration of NaOH in the solution of step d) is 0.1 to 4 mol/L.

6. Method according to one of the Claims 1 to 5, wherein the precipitation is performed at room temperature.

7. Method according to one of the Claims 1 to 6, comprises the further steps
f) Dissolve the isolated compound of step e) in water and lowering the pH of the aqueous solution to a value below pH 11 using an acid;
g) Isolation of a compound according to formula (I), wherein R₃ is hydrogen, from the solution of step f).

8. Method according to Claim 7, wherein in step f) the pH is adjusted to 4 to 7.

9. Method according to Claims 7 or 8, wherein in step f) the acid is HCl, p-TSA, acetic caid, H₃PO₄ or H₂SO₄.

10. A composition comprising a compound of formula (I) according to one of the preceding Claims.

11. Use of the composition of Claim 10 as a surfactant, antioxidant, UV-light absorber or stabilizing agent.

12. Use according to Claim 11, wherein the composition comprises 4-propylsyringol as a surfactant, UV light absorber or stabilizing agent.

13. Use according to Claim 11, wherein the composition comprises a 4-propylsyringol salt as a surfactant, antioxidant, UV light absorber or stabilizing agent.
